# EUROPÄISCHE PATENTSCHRIFT

(11) **EP 2 276 477 B1**
(45) Veröffentlichungstag und Bekanntmachung des Hinweises auf die Patenterteilung: **15.08.2018**
(21) Anmeldenummer: 09726650.6
(22) Anmeldetag: 26.03.2009
(51) Int. Cl.: A61K 9/70, A61P 29/00, B09B 3/00, A61K 31/485, A61P 25/36

(54) **SELBSTZERSTÖRENDES TRANSDERMALES THERAPEUTISCHES SYSTEM MIT VERBESSERTER FUNKTIONALITÄT UND WIRKSAMKEIT**
SELF-DESTRUCTING TRANSDERMAL THERAPEUTIC SYSTEM HAVING IMPROVED FUNCTIONALITY AND EFFICACY
SYSTÈME THÉRAPEUTIQUE TRANSDERMIQUE AUTODESTRUCTIBLE PLUS FONCTIONNEL ET PLUS EFFICACE

(30) Priorität: 02.04.2008 DE 102008016804
(43) Veröffentlichungstag der Anmeldung: 26.01.2011
(73) Patentinhaber: LTS LOHMANN Therapie-Systeme AG, 56626 Andernach (DE)
(72) Erfinder: MAIER, Stephan, 51371 Leverkusen (DE); WIRZ, Margit, 56751 Polch (DE)
(74) Vertreter: Schweitzer, Klaus
(86) Internationale Anmeldenummer: PCT/EP2009/002205
(87) Internationale Veröffentlichungsnummer: WO 2009/121514

(56) Entgegenhaltungen:
- WO-A2-2007/137732
- US-A1- 2005 163 717
- US-A1- 2007 166 233

## Beschreibung

Die Erfindung betrifft ein transdermales therapeutisches System (TTS) oder auch transdermales Pflaster genannt, das die inhärente Eigenschaft besitzt, dass es sich nach Gebrauch selbst zerstört. Das erfindungsgemäße TTS enthält einen therapeutischen Wirkstoff, aus der Gruppe der Schmerzmittel oder Narkotika, der aus dem System durch Diffusion an die Haut herangetragen und dann transdermal zu therapeutischen Zwecken appliziert wird.

Transdermale Applikationen mit den Wirkstoffen Buprenorphin und Fentanyl sind die Arzneiformen der Wahl zur Behandlung von chronischen Schmerzen in der Langzeittherapie. Durch die kontinuierliche Abgabe von solchen hochwirksamen Schmerzmitteln über die Haut wird ein Schmerzpatient kontinuierlich mit einer konstanten Dosis an Schmerzmittel versorgt, so dass Plasmaspitzen und Plasmatäler vermieden werden. Das hat den Vorteil, dass durch eine niedrige aber ausreichende Plasmakonzentration des Wirkstoffs sowohl Nebenwirkungen durch Überdosierungen, aber auch vermeidbare Schmerzzustände durch Unterversorgung, nicht auftreten. Dem Fachmann sind beispielsweise die Handelsprodukte Transtec®, aber auch Durogesic® oder Durogesic Smat bekannt, die sich schon seit längerer Zeit in der Schmerztherapie gut bewährt haben.

Der Nachteil der TTS in der Schmerztherapie besteht allerdings darin, dass zur Aufrechterhaltung des so genannten Konzentrationsgradienten und damit des für die Therapie gewünschten Plasmaspiegels des Wirkstoffs über die gesamte Zeitdauer der Applikation der TTS stets eine größere Vorratsmenge an Wirkstoff im TTS enthalten sein muss als tatsächlich an den Patienten abgegeben wird. Das hat zur Folge, dass getragene TTS ein Missbrauchspotential für z.B. Angehörige der Drogenszene darstellen. Diese Personengruppen sind durchaus in der Lage, getragene TTS zu sammeln und diese mit primitivsten Hilfsmitteln zu extrahieren, um so den noch vorhandenen, restlichen Wirkstoff zu gewinnen und ihn zur Befriedigung der Drogensucht missbräuchlich einzunehmen.

In der Vergangenheit hat es daher nicht an Versuchen gemangelt, diesen unkontrollierten Missbrauch dadurch zu unterbinden, dass Patienten geraten worden ist, getragene Pflaster zu zerschneiden und sie dann durch die Toilette der Kanalisation zuzuführen. Nachteilig an diesem Verfahren ist, dass weder Gesetzgeber noch Arzneimittelhersteller die Gewähr übernehmen können, dass diese Empfehlung von den Patienten auch zuverlässig befolgt wird, außerdem stellt die massenweise Entsorgung durch die Kanalisation ein nicht zu unterschätzendes Umweltproblem dar.

In der Folge wurden TTS entwickelt, die neben dem Wirkstoff auch einen Antagonisten enthielten (z.B. WO 2004/098576, WO 90/04965, WO 2004/037259). Dadurch sollte die oben beschriebene Gewinnung bzw. missbräuchliche Extraktion des schmerzstillenden Wirkstoffs aus gebrauchten TTS verhindert, zumindest aber deutlich erschwert werden. Diese Schutzmaßnahmen haben sich jedoch als nicht ausreichend zur Vermeidung von Medikamentenmissbrauch erwiesen, da nach wie vor mit relativ einfachen Mitteln der eigentliche Wirkstoff durch fraktionierte Fällung von dem Antagonisten getrennt werden kann.

Die WO 2007/137732 beschreibt ein TTS, das neben einem Wirkstoff noch ein davon abgetrenntes, den Wirkstoff unbrauchbar machendes Agens in einer Lösung enthält. Zusätzlich dazu ist noch ein Mittel vorhanden, das nach Gebrauch des TTS den Weg frei macht, damit das Agens mit dem Wirkstoff in Kontakt tritt und diesen unbrauchbar macht. Der Nachteil an dieser ansonsten perfekten Lösung besteht aber darin, dass das Agens in Lösung wegen seiner hohen Reaktivität die Lagerfähigkeit einschränkt und dass teilweise auch beim Transport die Gefahr von Beschädigung durch Flüssigkeitsaustritt vorhanden ist.

Der vorliegenden Erfindung lag daher die Aufgabe zu Grunde, ein TTS bereitzustellen, bei dem nach bestimmungsgemäßem Gebrauch eine missbräuchliche Entnahme des restlichen Wirkstoffs möglichst vollständig unmöglich bleibt und das zusätzlich über eine längere Zeitdauer problemlos lagerfähig ist und darüber hinaus Transportschäden durch unbeabsichtigten Austritt von in Flüssigkeit gelöstem Agens nicht vorkommen lässt.

Gelöst wird diese Aufgabe durch die Bereitstellung eines TTS gemäß Anspruch 1, bevorzugt in Form eines auf die Hautoberfläche des Patienten aufzubringenden transdermalen Pflasters, das sich nach Gebrauch, d.h. nach Abnahme des TTS von der Hautoberfläche des Patienten, selbst zerstört. Sich selbst zerstörendes TTS bedeutet anmeldungsgemäß, dass der in dem TTS enthaltene restliche Arzneimittelwirkstoff nach Gebrauch direkt oder indirekt zerstört, chemisch zersetzt und/oder unbrauchbar gemacht wird. Dabei ist aber auch immer gewährleistet, dass dieser Zerstörungsprozess nicht schon vor oder noch während der transdermalen Applikation des TTS gestartet wird.

Gegenstand der Erfindung ist somit ein transdermales therapeutisches System (TTS) gemäß Anspruch 1. Gegenstände, die nicht durch den Schutzumfang der Ansprüche umfasst sind, sind nicht Teil der beanspruchten Erfindung. Die mobile Phase bewirkt, dass das Agens aktiviert und in aktivierter Form mit dem Wirkstoff in Kontakt gebracht wird, der durch diesen Kontakt zersetzt, zerstört und so in seiner Wirksamkeit unbrauchbar gemacht wird.

Bei dem Agens handelt es sich um eine Substanz oder um ein Substanzgemisch, die oder das erfindungsgemäß als Feststoff oder als Paste vorliegt. Das Agens ist eine Substanz, die mit dem Wirkstoff chemisch reagiert und ihn dadurch zerstört, nämlich ein chemisches Oxidationsmittel wie z.B. anorganische Reagenzien, wie Permanganate, z.B. Kaliumpermanganat, Mangandioxid, Bleidioxid, Bleitetraacetat, Cer(IV)-Salze, Chromate, Osmiumtetroxid, Nitrite, wie Kaliumnitrit, Selendioxid, Peroxo-Verbindungen, Hypohalogenide, oder Schwefel; bevorzugt Kaliumpermanganat und Kaliumnitrit. Organische Oxidantien, wie Dimethylsulfoxid, N-Bromsuccinimid, Chinone, hypervalente lodverbindungen, Persäuren und Perester, aber auch Enzyme können zum Einsatz kommen. Bei gegebenem Wirkstoff wird das Agens bevorzugt auf Grund seiner chemischen Reaktionsfähigkeit mit dem Wirkstoff ausgewählt.
Bei dem Wirkstoff handelt es sich um einen Wirkstoff aus der Gruppe der Schmerzmittel wie z. B. Narkotika. Bevorzugt sind zu nennen Morphinderivate, Heroin und Buprenorphin, oder Fentanyl und seine Derivate Sufentanil und Alfentanyl. Grundsätzlich sind auch alle anderen Kombinationen von Wirkstoff und Agens verwendbar, für die eine transdermale Applikation über ein TTS eine geeignete Darreichungsform ist.

Die Trennung zwischen dem Wirkstoff und dem Agens wird durch eine für Flüssigkeiten durchlässige, für Feststoffe aber undurchlässige Schicht, zum Beispiel ein Papier, eine Membran oder ein Vlies bewirkt. Dabei kann das Vlies aus mineralischen Fasern, wie z.B. Glas, Mineralwolle, Basalt, tierischen Fasern wie z.B. Seide oder Wolle, pflanzlichen Fasern wie z.B. Baumwolle oder chemischen Fasern aus natürlichen (z.B. Cellulose) und/oder synthetischen Polymeren bestehen. Als synthetische Kunststoffe können hierzu Standardpolymere eingesetzt werden wie z.B. Polyamid, Polyimid, Polytetrafluorethylen, Polyethylen, Polypropylen, Polyvinylchlorid, Polyacrylate oder Polymethacrylate, Polystyrol, Polyester oder Polycarbonate.
Beim Abnehmen des Pflasters/TTS von der Haut des Patienten wird die Trennung zwischen Wirkstoff und Agens insofern aufgehoben, als ein Zutritt von Flüssigkeit an das Agens stattfindet oder zumindest möglich wird. Die Flüssigkeit tritt an das Agens heran, löst es auf, aktiviert es dabei und hilft so, dass das Agens durch zum Beispiel das Vlies hindurch bewegt wird, mit dem Wirkstoff in unmittelbaren Kontakt tritt und ihn dabei zerstört.

Das Mittel, das den Zutritt von Flüssigkeit bewirkt oder ermöglicht, ist ein mechanisches Mittel, das in unterschiedlichen Formen auftreten kann. Es soll damit auf jeden Fall gewährleistet sein, dass bei jeder Abnahme des TTS, und zwar unabhängig von der Abzugsrichtung, das Mittel seine bestimmungsgemäße Funktion erfüllt, nämlich direkt oder indirekt die Aufhebung der Trennung zwischen Wirkstoff und Agens zu ermöglichen, was aber zeitlich nicht schon vorher passieren darf. Dazu besitzt das Mittel eine Vielzahl von scharfen oder spitzen Bereichen. Einfachste nicht-erfindungsgemäße Form eines solchen Mittels ist ein Stern.

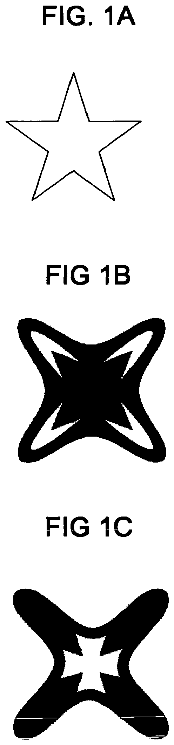

Ein Stern ist beispielhaft in **Figur 1A** dargestellt. Ein solcher Stern kann scharfe Spitzen, Dornen oder Kanten aufweisen, die ab einem bestimmten Biegeradius oder ab einer bestimmten mechanischen Beanspruchung des TTS zur Perforation mindestens einer benachbarten Schicht führen, die zum Beispiel eine Wand eines Flüssigkeitsvorrats oder eine Trennfolie sein kann, und so den Zutritt von Flüssigkeit bewirken oder zumindest ermöglichen.

Das mechanische Mittel zur Perforation besitzt erfindungsgemäß eine stumpfe Außenkontur und einen scharfen oder spitzen innenliegenden Bereich.

Beispiele für eine solche erfindungsgemäße Geometrie sind in **Figur 1B** und **Figur 1C** dargestellt. Beide Darstellungen zeigen Geometrien mit rundem, stumpfem und somit entschärftem äußerem Rand, die erfindungsgemäß eingesetzt werden. Damit besteht nämlich nicht länger die Gefahr, dass die Perforation unbeabsichtigt bei der Herstellung, bei der Lagerung, beim Transport oder während der bestimmungsgemäßen Handhabung vorzeitig passiert und somit der Wirkstoff schon vor seinem Gebrauch zerstört wird. Die spitzen, scharfen Bereiche liegen geschützt im Inneren der Geometrie. Bei großen Biegeradien und/oder bei geringer Krafteinwirkung auf das TTS wird somit die Perforation nicht eingeleitet. Erst beim Abziehen des TTS von der Haut ist der Biegeradius ausreichend klein oder es sind die zur Wirkung kommenden mechanischen Kräfte ausreichend groß um durch Verbiegen der Struktur um durch die Geometrie vorgegebene Drehpunkte die entsprechende Spitze im Innenbereich um einen Winkel bis maximal 90° aus der Ebene in Richtung zur benachbarten Schicht herauszudrehen. Auf Grund der durch die Steifigkeit des Materials erreichten Spannung im System wird eine Perforation mindestens einer benachbarten Schicht erreicht.

Dabei ist es besonders zweckmäßig, dass das mechanische Mittel, mit dem die Perforation mindestens einer benachbarten Schicht bewirkt wird, eine an die Flächenausdehnung des TTS angepasste Größe besitzt, vorzugsweise ist es nur geringfügig kleiner als die Innenfläche des TTS. Damit wird einerseits eine ausreichende Flexibilität des Systems gewährleistet, während andererseits die durch das Umbiegen erreichte Spannung in der Struktur für eine Perforation der benachbarten Schicht ausreicht. Daneben spielt auch das Verhältnis der Länge der Spitze zur Gesamtlänge des Mittels in Kraftrichtung eine Rolle. Je kürzer die Länge der Spitze ist, umso empfindlicher ist das System, da sich mit einer Verkürzung der Spitzenlänge deren Steifigkeit im Verhältnis zur Gesamtlänge des Mittels erhöht. Durch Krafteinwirkung wie Zug nach oben beim Abziehen des TTS wird die Spitze um ihren Drehpunkt/ihre Drehpunkte geschwenkt und dann in einem spitzen Winkel im Bereich von 20 bis 90° durch mindestens eine benachbarte Schicht gedrückt.

Als Material für das mechanische Mittel eignet sich zum Beispiel ein flexibler Kunststoff mit ausreichender Steifigkeit. Kunststoffe mit entsprechenden Eigenschaften sind zum Beispiel Standardpolymere wie Polyethylen oder Polypropylen, Polyester wie Polyethylentherephthalat, aber auch andere Polymere wie Cyclo-Olefin-Copolymere, Polyacrylate oder Polymethacrylate, Polytetrafluorethylen, PVC, Polycarbonat, Polylstyrol, Perfluoralkoxy, Perfluorethylenpropylen. Die Materialstärke beeinflusst die Wirksamkeit beim bestimmungsgemäßen Einsatz. Die Kunststoffe werden in Dicken von 100 bis 1000 µm, bevorzugt von 200 bis 700 µm, besonders bevorzugt von 250 bis 550 µm eingesetzt. Durch die bevorzugte Geometrie des Mittels, nämlich das Verhältnis der Länge der Spitze zur Gesamtlänge und durch die Anordnung der Drehpunkte wird trotz der Steifigkeit des Materials eine hohe Flexibilität und ein angenehmes Tragegefühl des TTS erreicht, ohne dass die Funktionalität der Selbstzerstörung verloren geht.

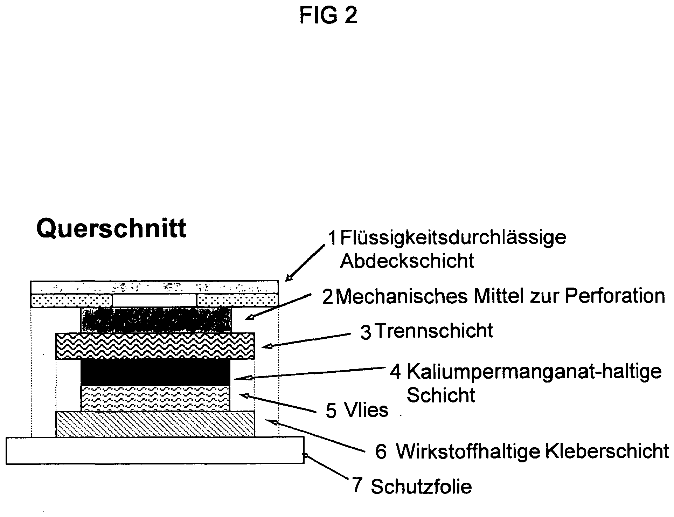

Das erfindungsgemäße TTS besitzt prinzipiell einen mehrschichtigen Aufbau, für den eine mögliche Variante in dem als Figur 2 beigefügten Ausführungsbeispiel exemplarisch erläutert wird.

Figur 2 zeigt einen senkrechten Schnitt durch ein erfindungsgemäßes TTS mit einem möglichen mehrschichtigen Aufbau. Dieser umfasst in der Darstellung mindestens eine z.B. flüssigkeitsdurchlässige obere Abdeckschicht 1 aus z.B. hautfarben eingefärbtem Gewebe, das auf seiner unteren Seite zumindest bereichsweise mit einer dünnen Kleberschicht überzogen ist, und eine untere Kleberschicht 6, die beim bestimmungsgemäßen Gebrauch des TTS in unmittelbarem Hautkontakt steht und in die der Wirkstoff eingelagert ist. Der Wirkstoff wird von dort aus in die oberste Schicht der Haut, die Epidermis, abgegeben.

Möglich ist auch die Ausbildung als Membranpflaster, bei dem zwischen einem Wirkstoff-Reservoir und der Haut eine klebende Membran angeordnet ist, die den Wirkstoff in die Epidermis abgibt und die in der Lage ist, die Geschwindigkeit der Abgabe zu steuern.

Zwischen der z.B. bereichsweise flüssigkeitsdurchlässig ausgebildeten oberen Abdeckschicht 1 und einer im Ausgangszustand z.B. für Flüssigkeiten undurchlässigen Trennschicht 3 befindet sich mindestens das mechanische Mittel zur Perforation 2. Unterhalb der Trennschicht 3 befindet sich ein Reservoir 4 für das Agens zur Zerstörung des Wirkstoffes, vorzugsweise ein Oxidationsmittel in fester Form, darunter ein Vlies 5 und darunter die vorstehend schon erwähnte Kleberschicht 6 mit dem Wirkstoff. Für Lagerung und Transport des TTS ist unterhalb der Kleberschicht 6 noch eine transparente Schutzfolie 7 vorgesehen, die vor Gebrauch des TTS zu entfernen ist.

In einer anderen bevorzugten Ausführungsform der Erfindung kann unterhalb der oberen Abdeckschicht 1 ein versiegelter Beutel mit einem Flüssigkeitsvorrat angeordnet sein, in dem sich auch das mechanische Mittel zur Perforation befinden kann.

Im Übrigen können für die Herstellung des erfindungsgemäßen TTS bzw. transdermalen Pflasters alle Materialien zum Einsatz kommen, die dem Fachmann für solche Systeme bekannt sind.
Für die Herstellung des erfindungsgemäßen TTS kann der Fachmann somit grundsätzlich auf die Materialien, Herstellungsverfahren und den Aufbau der aus dem Stand der Technik bekannten TTS bzw. transdermalen Pflaster zurückgreifen, die erfindungsgemäß zusätzlich eine geeignete Kombination aus Mittel und Agens aufweisen (vgl. dazu: Transdermale Pflaster; Spektrum der Wissenschaft 10/2003, 42; Transdermal Controlled Systemic Madications, Y.W. Chien, Drugs and the Pharmaceutical Sciences, Vol. 31; Polymers in Transdermal Drug Delivery Systems, S. Kandavilli et. al., Pharmaceutical Technology, May 2002, 62-80).

Voraussetzung für die Eignung von Kunststoffen für solche medizinischen Anwendungen ist neben günstigen Werkstoffeigenschaften wie mechanische Festigkeit, geringes Eigengewicht und ausreichend gute Verarbeitbarkeit aus hygienischen Gründen in erster Linie die gute Sterilisierbarkeit. Diese Anforderungen werden z.B. von Polyethylen, Polypropylen, Polyvinylchlorid, Polystyrol, Polymethacrylaten, Polyamiden, Polyestern und Polycarbonaten in ausreichender Weise erfüllt.

Die Erfindung wird durch die folgenden Beispiele näher erläutert ohne darauf beschränkt zu sein. Gleichwohl können spezielle, in den Beispielen beschriebene Ausgestaltungen des erfindungsgemäßen TTS einzeln oder in Kombination miteinander als bevorzugte Merkmale für die Erfindung als solche verallgemeinert werden.

### Beispiel 1

Zu 1,14 kg einer Lösung eines selbst vernetzenden Polyacrylates, bestehend aus den Monomeren 2-Ethylhexylacrylat, Vinylacetat, Butylacrylat und Acrylsäure in der Mischung der organischen Lösemittel Ethylacetat, Heptan und Isopropanol/Toluol, wurden 100 g Lävulinsäure, 150 g Oleyloleat, 100 g Polyvinylpyrrolidon, 150 g Ethanol, 200 g Ethylacetat und 100 g Buprenorphin Base zugegeben. Diese Mischung wurde bis zur Homogenisierung über eine Zeitdauer von etwa 2 Stunden gerührt. Nach dem Homogenisieren wurde die Mischung auf die silikonisierte Seite einer 100 µm Polyesterfolie aufgetragen, anschließend daran wurden die Lösemittel durch 10-minütiges Trocknen bei 70 °C im Trockenschrank entfernt. Die Strichstärke in der Beschichtung wurde so gewählt, dass nach Entfernen der Lösemittel ein Flächengewicht von ca. 80 g/m² resultierte. Nach dem Entfernen der Lösemittel wurde das Laminat bestehend aus silikonisierter Polyesterfolie und wirkstoffhaltiger Polymerschicht mit einer zweiten, schwächer silikonisierten Polyesterfolie abgedeckt. Danach wurde das so entstandene Laminat in Quadrate der Kantenlänge 5 x 5 cm zugeschnitten. Die 5 x 5 cm große silikonisierte Polyesterfolie wurde dann auf der einen Seite des Laminates entfernt und es wurde mittig ein zum Beispiel 4 x 4 cm großes saugfähiges, flüssigkeitsdurchlässiges Material, z.B. ein Vlies, aufgeklebt. Auf das saugfähige, flüssigkeitsdurchlässige Vlies wurde dann ein randseitig geprägter Beutel aus Filterpapier, gefüllt mit Kaliumpermanganat in Pulverform, aufgelegt, der so gestaltet war, dass er in seiner Gesamtfläche kleiner war als die wirkstoffhaltige Polymerschicht.

Ohne die Erfindung einzuschränken, kann der Beutel Maße von 4 x 4 cm aufweisen. Nun wurde auf den Beutel gefüllt mit Kaliumpermanganat eine für Flüssigkeiten undurchlässige Trennschicht in der Größe 5 x 5 cm aufgelegt und an den Rändern mit der wirkstoffhaltigen Polymerschicht verklebt. Dann wurde ein vierzackiger Malteser in der Art, wie er in Figur 1C dargestellt ist, aus hartem Polymermaterial aufgelegt. In einem nachfolgenden Arbeitsschritt wurde dann noch eine flüssigkeitsdurchlässige obere Abdeckfolie in Form eines Laminates, bestehend aus einer bereichsweise aufgebrachten wirkstofffreien Haftkleberschicht und hautfarben eingefärbtem Gewebe, Dicke = 21 µm, so aufgeklebt, dass die wirkstofffreie Haftkleberschicht die wirkstoffhaltige Polymerschicht allseitig am Rand überragt. Schließlich wurde die noch vorhandene zweite silikonisierte Polyesterfolie der Größe 5 x 5 cm von der wirkstoffhaltigen Polymerschicht entfernt und durch eine Schutzfolie mit den gleichen Abmessungen wie die der oberen Abdeckfolie ersetzt.

Wird das TTS im Rahmen seines bestimmungsgemäßen Verwendungszweckes appliziert, muss zunächst einmal die silikonisierte Polyesterschicht (Schutzfolie) entfernt werden, was leicht möglich ist. Wird das TTS auf die Haut eines Patienten aufgeklebt, dann bleibt die für Flüssigkeiten undurchlässige Trennschicht zunächst unbeschädigt. Ein Zutritt von Flüssigkeit zum Kaliumpermanganatpulver ist nicht möglich. Wird aber nach der Applikationsdauer von 1 bis 7 Tagen das TTS von der Haut des Patienten abgezogen, dann bohrt sich mindestens eine Spitze des vierzackigen Maltesers auf Grund der Steifigkeit des Polymermaterials durch die Trennschicht und perforiert diese zwangsläufig. Durch die Geometrie des Maltesers ist sichergestellt, dass die Trennschicht in jedem Falle perforiert wird, egal in welcher Richtung das TTS vom Patienten entfernt wird.

Wenn dann das gebrauchte TTS in Wasser gelegt wird, kann das Wasser durch die Abdeckfolie und die Perforation in der Trennschicht in das TTS eindringen, das Kaliumpermanganat auflösen und durch das saugfähige Vlies hindurch innerhalb kurzer Zeit zu dem restlichen Wirkstoff in der unteren Kleberschicht hintransportieren. Dort wird sofort ein Oxydationsprozess gestartet, der im Falle von z.B. Buprenorphin zu dessen oxydativer Zerstörung führt. Durch Einlegen des gebrauchten TTS in Wasser ist so sichergestellt, dass der Wirkstoff nicht missbraucht werden kann.

### Beispiel 2

Beispiel 1 wurde wiederholt mit dem Unterschied, dass zwischen die obere Abdeckschicht und den Beutel mit dem Kaliumpermanganat in Pulverform ein rundum versiegelter, flüssigkeitsdichter Beutel gefüllt mit dem Malteser und einer Menge von 1,5 ml Wasser eingeklebt wurde. Beim Abziehen des gebrauchten TTS von der Haut des Patienten bohrt sich mindestens eine Spitze des Maltesers durch die untere Wand des Flüssigkeitsbeutels und bewirkt so den Austritt des Wassers, das mit dem darunter angeordneten Kaliumpermanganat sofort in Kontakt tritt.

In dieser Ausführungsform der Erfindung braucht der Patient das gebrauchte TTS nicht in Wasser zu legen, um den Zerstörungsvorgang einzuleiten, sondern es zerstört sich automatisch von selbst beim Abziehen nach Gebrauch.

## Patentansprüche

1. Selbstzerstörendes transdermales therapeutisches System (TTS) enthaltend
- mindestens einen Wirkstoff, welcher ein Schmerzmittel oder Narkotikum ist,
- mindestens ein den Wirkstoff unbrauchbar machendes Agens, welches ein Oxidationsmittel ist und als Feststoff oder Paste vorliegt,
- mindestens eine Trennung zwischen dem Wirkstoff und dem den Wirkstoff unbrauchbar machenden Agens, wobei die Trennung eine Schicht ist, die für Flüssigkeiten durchlässig und für Feststoffe undurchlässig ist und
- mindestens ein mechanisches Mittel zur Perforation mindestens einer benachbarten Schicht des TTS, wobei das Mittel zur Perforation eine stumpfe Außenkontur und einen scharfen oder spitzen innenliegenden Bereich besitzt und welches oberhalb einer für Flüssigkeiten undurchlässigen Trennschicht angeordnet ist,
wobei das Mittel zu Perforation bewirkt, dass beim Abziehen des TTS nach Gebrauch mindestens eine benachbarte Schicht des TTS perforiert wird, und der Zutritt einer mobilen Phase, welche eine Flüssigkeit ist, an das den Wirkstoff unbrauchbar machende Agens bewirkt oder ermöglicht wird, wodurch die Trennung zwischen dem Wirkstoff und dem den Wirkstoff unbrauchbar machenden Agens aufgehoben wird, dass so der Wirkstoff und das den Wirkstoff unbrauchbar machende Agens miteinander in Berührung kommen und durch diesen Kontakt der Wirkstoff zerstört wird.

2. Selbstzerstörendes transdermales therapeutisches System nach Anspruch 1, **dadurch gekennzeichnet, dass** der Wirkstoff und das den Wirkstoff unbrauchbar machende Agens nach dem Abziehen des TTS von der Haut des Patienten in Anwesenheit einer mobilen Phase chemisch miteinander reagieren.

3. Selbstzerstörendes transdermales therapeutisches System nach Anspruch 1 oder 2, **dadurch gekennzeichnet, dass** der Wirkstoff ein Morphinderivat, Heroin oder Buprenorphin ist.

4. Selbstzerstörendes transdermales therapeutisches System nach einem oder nach mehreren der Ansprüche 1 bis 3, **dadurch gekennzeichnet, dass** der Wirkstoff Fentanyl, Sufentanil oder Alfentanyl ist.

5. Selbstzerstörendes transdermales therapeutisches System nach einem oder nach mehreren der Ansprüche 1 bis 4, **dadurch gekennzeichnet, dass** das den Wirkstoff unbrauchbar machende Agens Kaliumpermanganat ist.

6. Selbstzerstörendes transdermales therapeutisches System nach einem oder nach mehreren der Ansprüche 1 bis 5, **dadurch gekennzeichnet, dass** die mobile Phase Wasser ist.

7. Selbstzerstörendes transdermales therapeutisches System nach einem oder nach mehreren der Ansprüche 1 bis 6, **dadurch gekennzeichnet, dass** das mechanische Mittel zur Perforation eine Vielzahl von scharfen oder spitzen Bereichen besitzt.

8. Selbstzerstörendes transdermales therapeutisches System nach einem oder nach mehreren der Ansprüche 1 bis 7, **dadurch gekennzeichnet, dass** das mechanische Mittel zur Perforation eine sternförmige Form besitzt.

9. Selbstzerstörendes transdermales therapeutisches System nach Anspruch 1, **dadurch gekennzeichnet, dass** bei dem mechanischen Mittel zur Perforation beim Abziehen des TTS von der Haut durch Verbiegen seiner Struktur um durch ihre Geometrie vorgegebene Drehpunkte mindestens eine entsprechend angeordnete Spitze im Innenbereich um einen Winkel bis maximal 90° aus der Ebene in Richtung zur benachbarten Schicht herausgedreht wird und so eine Perforation mindestens einer benachbarten Schicht bewirkt.

## Claims

1. Self-destructing transdermal therapeutic system (TTS) comprising
- at least one active ingredient which is an analgesic or narcotic,
- at least one agent which makes the active ingredient useless and which is an oxidizing agent and is present as a solid or paste,
- at least one separation between the active ingredient and the agent which makes the active ingredient useless, wherein the separation is a layer which is permeable to liquids and impermeable to solids, and
- at least one mechanical means for perforation of at least one adjacent layer of the TTS, wherein the means for perforation possesses a blunt outer contour and a sharp or pointed internal region, and which means is arranged above a separating layer impermeable to liquids,
wherein by the means for perforation at least one adjacent layer of the TTS is perforated on removal of the TTS after use, and the ingress of a mobile phase, which is a liquid, to the agent which makes the active ingredient useless is effected or made possible, whereby the separation between the active ingredient and the agent which makes the active ingredient useless is undone, that in this way the active ingredient and the agent which makes the active ingredient useless come into contact with one another, and the active ingredient is destroyed by this contact.

2. Self-destructing transdermal therapeutic system according to Claim 1, **characterized in that** the active ingredient and the agent which makes the active ingredient useless react chemically with one another in the presence of a mobile phase following removal of the TTS from the patient's skin.

3. Self-destructing transdermal therapeutic system according to Claim 1 or 2, **characterized in that** the active ingredient is a morphine derivative, heroin or buprenorphine.

4. Self-destructing transdermal therapeutic system according to one or more of Claims 1 to 3, **characterized in that** the active ingredient is fentanyl, sufentanil or alfentanyl.

5. Self-destructing transdermal therapeutic system according to one or more of Claims 1 to 4, **characterized in that** the agent which makes the active ingredient useless is potassium permanganate.

6. Self-destructing transdermal therapeutic system according to one or more of Claims 1 to 5, **characterized in that** the mobile phase is water.

7. Self-destructing transdermal therapeutic system according to one or more of Claims 1 to 6, **characterized in that** the mechanical means for perforation possesses a multiplicity of sharp or pointed regions.

8. Self-destructing transdermal therapeutic system according to one or more of Claims 1 to 7, **characterized in that** the mechanical means for perforation possesses a stellate shape.

9. Self-destructing transdermal therapeutic system according to Claim 1, **characterized in that**, with regard to the mechanical means for perforation, on removal of the TTS from the skin, by distortive bending of its structure, at least one correspondingly arranged point in the inner region is rotated, around pivots dictated by its geometry, by an angle of up to a maximum 90° out of the plane in the direction of the adjacent layer and in this way perforation of at least one adjacent layer is effected.

## Revendications

1. Système thérapeutique transdermique (TTS) autodestructible contenant
- au moins un principe actif, qui est un analgésique ou un narcotique,
- au moins un agent qui rend le principe actif inutilisable, lequel est un oxydant et est présent en tant que substance solide ou pâte,
- au moins une séparation entre le principe actif et l'agent qui rend le principe actif inutilisable, dans lequel la séparation est une couche, qui est perméable aux fluides et imperméable aux substances solides et
- au moins un moyen mécanique pour la perforation d'au moins une couche voisine du TTS, dans lequel le moyen de perforation dispose d'un contour extérieur émoussé et d'une zone intérieure tranchante ou pointue et lequel est agencé au-dessus d'une couche de séparation imperméable aux fluides,
dans lequel le moyen de perforation a pour effet que lors du retrait du TTS après utilisation au moins une couche voisine du TTS est perforée, et l'accès d'une phase mobile, laquelle est un fluide, à l'agent qui rend le principe actif inutilisable est entraîné ou rendu possible, moyennant quoi la séparation entre le principe actif et l'agent qui rend le principe actif inutilisable est supprimée, que le principe actif et l'agent qui rend le principe actif inutilisable entrent ainsi en contact l'un avec l'autre et le principe actif est détruit par ce contact.

2. Système thérapeutique transdermique autodestructible selon la revendication 1, **caractérisé en ce que** le principe actif et l'agent qui rend le principe actif inutilisable réagissent chimiquement l'un avec l'autre après le retrait du TTS de la peau du patient en l'absence d'une phase mobile.

3. Système thérapeutique transdermique autodestructible selon la revendication 1 ou 2, **caractérisé en ce que** le principe actif est un dérivé de morphine, de l'héroïne ou de la buprénorphine.

4. Système thérapeutique transdermique autodestructible selon une ou plusieurs des revendications 1 à 3, **caractérisé en ce que** le principe actif est du fentanyl, sufentanil ou alfentanil.

5. Système thérapeutique transdermique autodestructible selon une ou plusieurs des revendications 1 à 4, **caractérisé en ce que** l'agent qui rend le principe actif inutilisable est du permanganate de potassium.

6. Système thérapeutique transdermique autodestructible selon une ou plusieurs des revendications 1 à 5, **caractérisé en ce que** la phase mobile est de l'eau.

7. Système thérapeutique transdermique autodestructible selon une ou plusieurs des revendications 1 à 6, **caractérisé en ce que** le moyen mécanique de perforation dispose d'une pluralité de zones tranchantes ou pointues.

8. Système thérapeutique transdermique autodestructible selon une ou plusieurs des revendications 1 à 7, **caractérisé en ce que** le moyen mécanique de perforation a une forme en étoile.

9. Système thérapeutique transdermique autodestructible selon la revendication 1, **caractérisé en ce que** dans le moyen mécanique de perforation lors du retrait du TTS de la peau par déformation de sa structure autour de points de rotation prédéfinis par leur géométrie, au moins une pointe agencée en conséquence est dévissée dans la zone intérieure d'un angle allant jusqu'à 90° maximum du plan en direction de la couche voisine et entraîne ainsi une perforation d'au moins une couche voisine.
